# EUROPEAN PATENT APPLICATION

(11) **EP 4 155 889 A1**
(43) Date of publication of application: **29.03.2023**
(21) Application number: 21306342.3
(22) Date of filing: 28.09.2021
(51) Int. Cl.: G06F 3/04845, G06F 3/01, G06F 3/04883

(54) **METHODS AND SYSTEMS FOR PERSONALIZED COLORING TEMPLATE**

(71) Applicant: Société BIC, 92110 Clichy (FR)
(72) Inventor: Duffy, David, 8003 Zurich (CH); Elliott-Bowman, Bernadette, Leatherhead, Surrey, KT22 8LR (GB); Cronin, Harry Michael, Cambridge, CB1 3DE (GB)
(74) Representative: Peterreins Schley

(57) **Abstract**

The present invention relates to a computer-implemented method for generating a personalized coloring template for a user of a user monitoring device, comprising obtaining user data generated by monitoring the user's emotional state via the user monitoring device; obtaining an emotion event generated by detecting an excited level of an emotion of the user based on the user data; and generating a coloring template based on the emotion event, thereby generating the personalized coloring template for the user.

The present invention further relates to a computer system configured to execute the computer-implemented method for generating a personalized coloring template for a user.

## Description

### TECHNICAL FIELD

This specification relates to a computer-implemented method and/or a computer system for generating a personalized coloring template for a user of a user monitoring device.

### BACKGROUND

Self-reflection is an important part of mindfulness and involves for a person taking time to reflect on things she/he has experienced. Personalized self-reflection imagery can be used to increase user engagement with and effectiveness of self-reflection by helping users to focus on her/his specific issues. Mindful coloring is popular for adults and other mindfulness, diary and life tracking apps are commonly used by adults for stress and health management.

As an example, emotions and physiological responses may be associated with colors. Furthermore, the sounds made by different types of stationery, as well as the type of brush/pen stroke may be used by autonomous sensory meridian response (ASMR) practitioners to reduce stress. Art therapy has also been shown to produce measurable biofeedback responses that indicate stress reduction. Such physiological responses may be tracked by wearables and/or other devices.

Artificial intelligence (AI) may be used to generate realistic or stylized faces, scenes and/or objects. As an example, deep-fake systems may transplant a user's face into movie scenes.

### SUMMARY

According to a first aspect, there is provided a computer-implemented method for generating a personalized coloring template for a user of a user monitoring device. The method may comprise obtaining user data generated by monitoring the user's emotional state via the user monitoring device. The method may further comprise obtaining an emotion event generated by detecting an excited level of an emotion of the user based on the user data. The method comprises generating a coloring template based on the emotion event, thereby generating the personalized coloring template for the user.

According to a second aspect, there is provided a computer system configured to execute the computer-implemented method of the first aspect (or an embodiment thereof) for generating a personalized coloring template for a user.

According to a third aspect, there is provided a computer program configured to execute the computer-implemented method of the first aspect (or an embodiment thereof) for generating a personalized coloring template for a user.

According to a fourth aspect, there is provided a computer-readable medium or signal storing the computer program of the third aspect (or an embodiment thereof).

Dependent embodiments of the aforementioned aspects are given in the dependent claims and explained in the following description, to which the reader should now refer.

Methods and/or systems of the aforementioned aspects of this specification are directed to generating personalized coloring templates for users of user monitoring devices.

Typically, or conventionally, people may find it difficult to remember and/or reflect on their own experiences. Some existing self-reflection and mindfulness products and services incorporate drawing and/or coloring activities such as e.g. adult coloring books. However, such products and services are not personalized to individual experiences of a user. As a consequence, they are limited in their effectiveness for self-reflection.

As disclosed hereinafter, personalized self-reflection coloring scenes may be designed based on user emotions and events, wherein the image complexity, user interaction and/or types of features presented in parts of the scene may be adapted to encourage focus on a particular feature and/or encourage self-reflection on a certain emotion and/or for a certain period of time.

User events and associated emotions may be gathered by, for example, correlating heart rate information from a wearable device with user location or schedule, accessing digital diary information, or other means.

In an embodiment, real time biofeedback from the user while coloring e.g. sections of the coloring template under guidance from e.g. a digital assistant is used to learn optimum colors, stroke style and/or features for personalized stress reduction. Such results may be used to adapt the properties of the current and/or subsequent personalized coloring template/scene.

An example scenario for the computer-implemented method of the first aspect (or an embodiment thereof) may be as follows: A user's emotional state while carrying out daily activities and/or interactions may be monitored by wearables and/or other devices. When the user is detected to be experiencing a stronger and/or more negative emotion than usual, the computer system of the second aspect (or an embodiment thereof) may use various devices and/or data sources to collect information about the event that is causing it. Subsequently, the event (or features of the event) may be algorithmically summarized and recreated in a generated coloring template. The template may be colored digitally or printed out. The layout and segmentation of the coloring template may be designed so that the user is encouraged to spend more time coloring in features of the image that have emotional, mindful and/or thematic significance. In so doing, the user may be encouraged to mindfully reflect on the event by coloring in the coloring template. The user may be monitored during the coloring experience to determine how it is affecting her/his stress state and generate guidance to improve it. A digital coloring template may be adjusted during the coloring session to alter the user's focus and/or stress state.

An example computer system of the second aspect (or an embodiment thereof) may be a system configured to generate personalized coloring templates that reflect a user's daily experiences, wherein selection of events to be reconstructed may be based on the user's emotional response during that event. The computer system may further be configured to correlate a user's emotional response to an event, such that details of the emotion and event may be used to define image-forming instructions for an image generation algorithm. The computer system may further be configured to adjust the layout of a coloring template such that the density and distribution of coloring segments may be adapted to focus the user's attention on certain features of the image. The computer system may further be configured to learn a user's optimum coloring experience for stress reduction, mindfulness and/or other desirable states based on the user's physiological/emotional responses to the various features of current and previous coloring templates. The computer system may further be configured to dynamically generate guidance for a user during coloring based on the user's emotional state and coloring behaviors. The computer system may further be configured to dynamically adjust the properties of a coloring template during coloring based on the user's emotional state and coloring behaviors.

Methods and systems of this specification are configured to generate personalized coloring templates that are intended to help a user mindfully reflect on her/his emotions and the specific experiences that induced them. In so doing, events and emotions from a user's daily experience can be captured and incorporated into a coloring template, enabling personalized coloring experiences that may enhance a person's ability to self-reflect. The coloring template generated by the method of the first aspect (or an embodiment thereof) may thus function as an emotional mirror. Furthermore, the effect of a mindful coloring experience on a user may be monitored to identify features, techniques and/or scenes that are particularly effective in helping the user to relax and/or relieve stress. Furthermore, features of the coloring scene may be customized to increase user focus on those areas, encouraging the user to reflect on specific objects, people and/or events more closely. Furthermore, personalized coloring templates may be colored in physically (that is on paper) or digitally, allowing the user the choice to mindfully color in a way that does not require them to use a device while still retaining a link between the coloring template and her/his experiences. As a result, the user's mindfulness and overall happiness may be increased.

### FIGURE DESCRIPTION

**Fig. 1a** schematically illustrates a computer-implemented method for generating a personalized coloring template for a user of a user monitoring device.
**Fig. 1b** is a continuation of the schematic illustration of the computer-implemented method for generating a personalized coloring template for a user of a user monitoring device.
**Fig. 2** schematically illustrates an example embodiment of the computer-implemented method for generating a personalized coloring template for a user of a user monitoring device.
**Fig. 3** illustrates an example architecture of a computer system configured to run the computer-implemented method for generating a personalized coloring template for a user of a user monitoring device.
**Fig. 4** illustrates an example flow chart for a computer system configured to run the computer-implemented method for generating a personalized coloring template for a user of a user monitoring device.
**Fig. 5** illustrates a coloring template.

### DETAILED DESCRIPTION

First, a method 100 for generating personalized mindful coloring templates is described. The method 100 may be designed to help a user mindfully reflect on her/his experiences and/or reduce stress by encouraging them to focus on certain features of the template that may have caused stress or other emotions.

There is disclosed a computer-implemented method 100 for generating a personalized coloring template for a user of a user monitoring device. The method 100 may comprise obtaining 111 user data generated by monitoring 110 the user's emotional state via the user monitoring device. The method 100 may further comprise obtaining 121 an emotion event generated by detecting 120 an excited level of an emotion of the user (at least) based on the user data. The method 100 further comprises generating 140 a coloring template 10 (at least) based on the emotion event, thereby generating the personalized coloring template for the user.

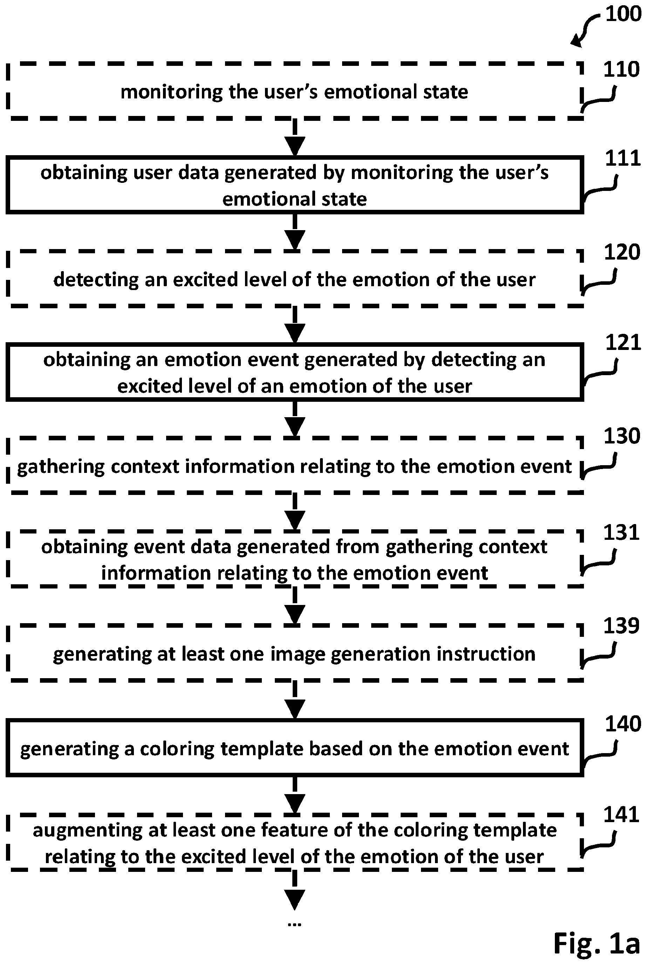

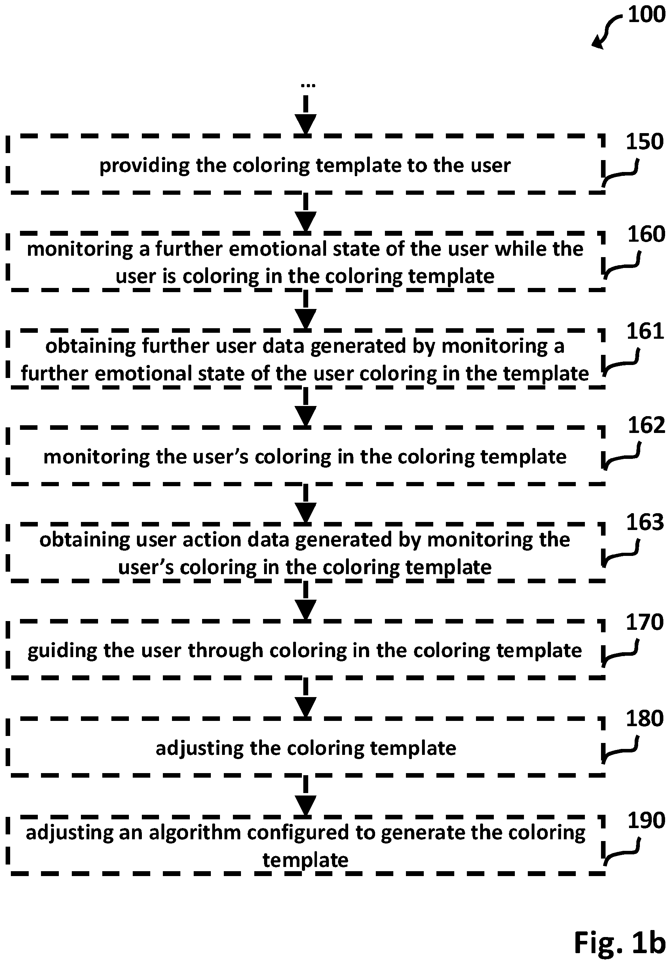

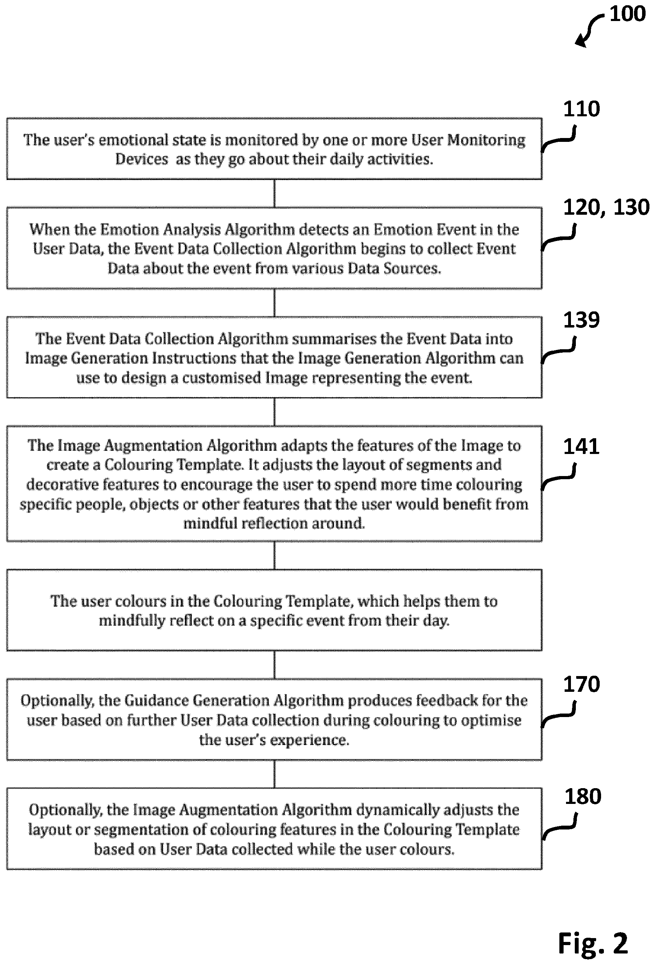

The computer-implemented method 100 is schematically illustrated in **Fig. 1a****-b** (with **Fig. 1b** being a continuation of **Fig. 1a**). In general, the order of steps in **Fig. 1a****-b** shall not be construed as limiting. For instance, the order of steps 170 and 180 is immaterial. An example scenario/embodiment of the method 100 is schematically illustrates in **Fig. 2****.**

The coloring template 10 may be a digital image. The digital image may be represented as a bit or byte sequence and, in particular, as a bitmap or a vector graphics. The coloring template 10 may be configured to encourage the user to mindfully reflect on the emotion event by coloring in the coloring template 10. Alternatively, or in addition, the coloring template 10 may be configured to encourage the user to focus on a (particular) feature of the coloring template 10. Alternatively, or in addition, the coloring template 10 may be configured to assist the user in relaxing and/or reducing stress.

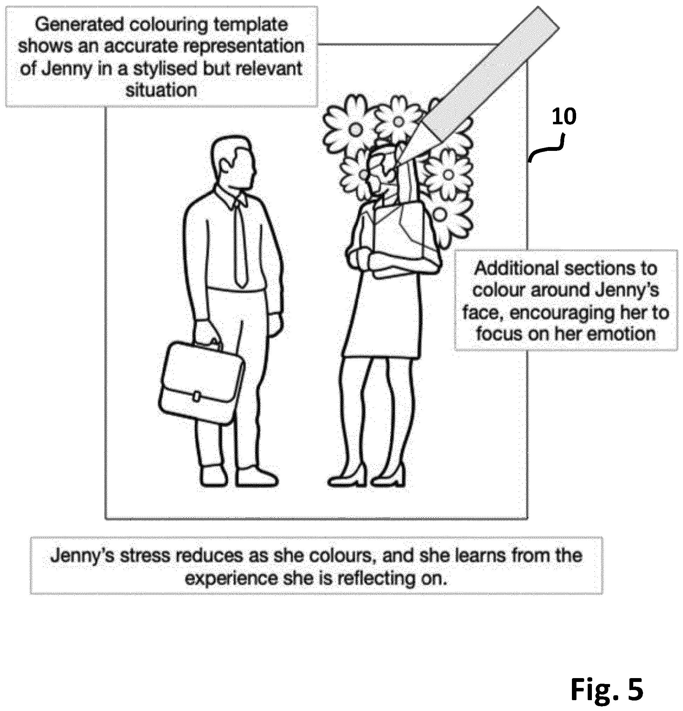

The coloring template 10 may, as e.g. displayed in **Fig. 5****,** comprise line art consisting of distinct straight and/or curved lines placed against a background, e.g. without gradations in shade or in hue to represent two-dimensional or three-dimensional objects. Lines may be monochromatic or of different colors. Lines may be contours defining bounded regions to be filled with a color. Filling in such regions may be referred to as coloring in the coloring template 10 (or segments thereof). The layout and/or segmentation of the coloring template 10 may be designed so that the user is encouraged to spend more time coloring in features of the image that have emotional, mindful and/or thematic significance. In so doing, the effect of the coloring template 10 on the user may be influenced and/or controlled.

The coloring template 10 may be printed on a sheet of paper via a user interface. Alternatively, or in addition, the coloring template 10 may be configured to be colored in by a user on a screen (e.g. on a smart device).

The method 100 may or may not comprise monitoring 110 the user's emotional state via the user monitoring device. If the method 100 comprises monitoring 110 the user's emotional state via the user monitoring device, steps 110, 111 may be combined into a single step 110 (unlike in **Fig. 1a**): monitoring 110 the user's emotional state via the user monitoring device, thereby generating user data. The user may be monitored 110 while carrying out daily activities and interactions. In this case, the coloring template for the user may be based on her/his daily experiences and associated emotional state.

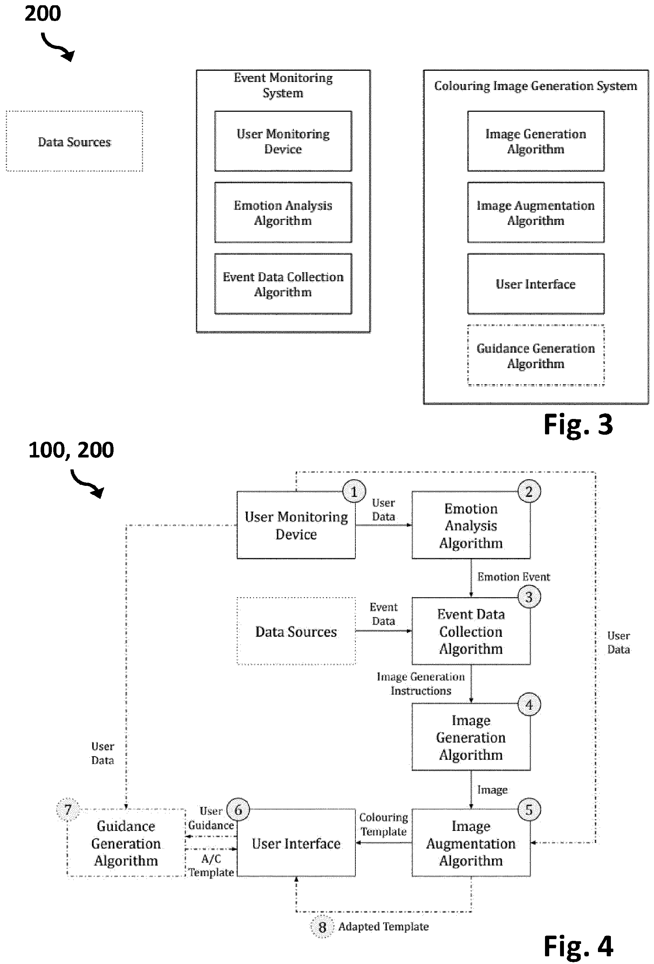

The method 100 may or may not comprise detecting 120 the excited level of the emotion of the user based on the user data. If the method 100 comprises detecting 120 the excited level of the emotion of the user based on the user data, steps 120, 121 may be combined into a single step 120 (unlike in **Fig. 1a**): detecting 120 an excited level of an emotion of the user based on the user data, thereby generating an emotion event. As an example, and as in **Fig. 3-4****,** detecting 120 the excited level of the emotion of the user based on the user data may be carried out by an emotion analysis algorithm.

As an example, a user (of the event monitoring system and/or of the coloring image generation system) may go about her/his day carrying out tasks and/or interacting with other people. One or more user monitoring devices may record the user's emotional state via the user data while the user carries out her/his various activities. Emotional responses may be determined by monitoring user data. For example, user data (or below: further user data) may comprise a heart rate of the user. Alternatively, or in addition, user data may comprise a galvanic skin response of the user. Alternatively, or in addition, user data may comprise an electroencephalography (EEG) of the user. Alternatively, or in addition, user data may comprise data relating to one or more facial expressions/micro-expressions of the user. Detecting 120 the excited level of the emotion of the user based on the user data may comprise analyzing such user data. In fact, for example, one or more (temporal) changes in the heart rate of the user, in the galvanic skin response of the user, in the EEG of the user, and/or in the one or more facial expressions/micro-expressions of the user, may serve as indicator(s) for an "excited" level of emotion. The user monitoring devices may take the form of devices comprising a smart device such as e.g. a smartphone or a tablet. Alternatively, or in addition, the user monitoring devices may take the form of devices comprising a wearable device such as e.g. a smartwatch, on-skin electronics and the like. Alternatively, or in addition, the user monitoring devices may take the form of devices comprising earbuds or other hearables. Alternatively, or in addition, the user monitoring devices may take the form of devices comprising smart glasses. Alternatively, or in addition, the user monitoring devices may take the form of devices comprising a smart headset such as e.g. brain-monitoring headwear. The one or more user monitoring devices are likely to be enabled by devices that can be carried with the user during their day.

Detecting 120 the excited level of the emotion of the user may comprise applying the user data or a portion thereof relating to a current point in time to a predetermined criterion. Detecting 120 the excited level of the emotion of the user may further comprise detecting the excited level of the emotion of the user relating to the current point in time, if the predetermined criterion is satisfied. The emotion event may comprise the current point in time. The emotion event may further comprise information specifying the emotion that has been detected. The emotion event may comprise a portion of the user data (relating to the current point in time). In examples, applying the user data (or a portion thereof) to the predetermined criterion may comprise computing a scalar quantity based on the user data (or a portion thereof) and checking, whether a pre-determined threshold value is exceeded, thereby defining the notion of an excited level of an emotion of the user. In examples, the predetermined criterion may be any classification algorithm configured to classify an excited level of an emotion of the user and, in particular, a machine learning algorithm that has been pre-trained based on training data. Such training data may comprise previous user data (and corresponding labels). The classification algorithm may be a multi-classification algorithm to recognize different emotions such as happiness, anger, envy, stress, anxiety etc. As e.g. in **Fig. 3-4****,** the predetermined criterion may be implemented in terms of the emotion analysis algorithm.

For example, the excited level of the emotion of the user may be detected 120 if one or more of the heart rate of the user, the galvanic skin response of the user, the electroencephalography of the user, and/or the facial expression(s)/micro-expression(s) of the user satisfy the predetermined criterion.

In examples, the emotion analysis algorithm may be configured to analyze the user data to detect one or more emotion events. Emotion detection techniques that are known in the art may be used to enable the emotion analysis algorithm. Arbitrary thresholds may be applied to user data to constrain the number or type of emotion events that are detected. For example, an emotion event may be categorized as such when the emotion experienced is algorithmically defined as intense/strong, that is for example, when the emotion surpasses a predetermined threshold specific to the physiological indicator being analyzed. In examples, an emotion event may be categorized as such when the emotion experienced is negative. The user may benefit from mindfully reflecting around such an emotion. The emotion event may be assigned a timestamp (relating to the current point in time) in order to facilitate data collection by subsequent system components.

Generating 140 the coloring template 10 based on the emotion event may comprise applying the emotion event (and e.g. event data etc.) to an algorithm, in particular, a machine-learning algorithm, that may be configured to and pre-trained to generate an image that mirrors the emotion event. The algorithm may be parametrized by user-defined settings that may e.g. be specified via a user interface of the computer system 200. In examples, and as in **Fig. 3-4****,** generating 140 the coloring template 10 based on the emotion event may be carried out by an image generation algorithm. The method 100 may comprise saving the coloring template 10 (e.g. in a storage of the computer system 200).

The method 100 may comprise obtaining 131 event data generated from gathering 130 context information relating to the emotion event. Generating 140 the coloring template 10 may further be based on the event data. Such event data (e.g. photo or video footage, soundtrack etc.) may be beneficial to better or fully capture the situation in which the user experiences the excited level of the emotion.

The method 100 may or may not comprise gathering 130 the context information relating to the emotion event. If the method 100 comprises gathering 130 the context information relating to the emotion event, steps 130, 131 may be combined into a single step (unlike in **Fig. 1a**): gathering 130 context information relating to the emotion event, thereby generating event data. As an example, and as in **Fig. 3-4****,** gathering 130 the context information relating to the emotion event may be carried out by an event data collection algorithm.

Gathering 130 the context information relating to the emotion event may comprise collecting context information relating to the emotion event via the user monitoring device. As an example, gathering 130 the context information relating to the emotion event may comprise collecting buffered context information relating to the emotion event via the user monitoring device. Alternatively, or in addition, gathering 130 the context information relating to the emotion event may comprise recording context information relating to the emotion event by at least one sensor system (e.g. other than the user monitoring device). For example, the at least one sensor system may comprise (or be) a camera system. Alternatively, or in addition, the at least one sensor system may comprise (or be) a microphone. Alternatively, or in addition, the at least one sensor system may comprise (or be) a GPS (or other navigation) sensor system. Alternatively, or in addition, gathering 130 the context information relating to the emotion event may comprise requesting context information from a server.

The method 100 may comprise generating 139 at least one image generation instruction based on the emotion event. Alternatively, or in addition, the method 100 may comprise generating 139 at least one image generation instruction based on the event data. Generating 140 the coloring template 10 may then be further based on the at least one image generation instruction.

As an example, the event data collection algorithm may use the timestamped emotion event to collect and summarize event data related to the event. When an emotion event is detected, the event data collection algorithm may request event data from a range of data sources e.g. in real time or with negligible time lag. For example, the microphone on a smartwatch, smartphone or other device may be activated and used to record a soundtrack of the emotion event, thereby generating event data. Alternatively, or in addition, the camera on a pair of smart glasses, a smartphone or other device may be activated and used to record photo or video footage of the emotion event, thereby generating event data. Alternatively, or in addition, Bluetooth or other short-range wireless technologies on a user's device may be activated to ping and identify nearby devices of other users in order to determine who the user is with, thereby generating event data. Instead of activating the at least one sensor system upon detecting 120 the excited level of the emotion of the user, it may be beneficial to continuously run the at least one sensor system and store data therefrom in a (ring) buffer. In so doing, recent data from the at least one sensor system may be captured during the emotion event (rather than shortly afterwards). Alternatively, or in addition, data from motion and/or other sensors in a smartwatch, smartphone or other device that may indicate the activity a user is engaged in may be collected, thereby generating event data. Alternatively, or in addition a user's GPS location may be determined, thereby generating event data.

Given the timestamp applied to the emotion event, the event data collection algorithm may also search other data sources to collect relevant event data at some time after the emotion event has occurred. For example, online sources to match a GPS location collected at the time of the emotion event may be utilized to map location and associated public images. Alternatively, or in addition, a user's digital journal or diary may be accessed. Alternatively, or in addition, a user's digital calendar may be accessed. Alternatively, or in addition, a user's activity tracking apps (such as e.g. Strava) may be accessed. Any such data may also generate event data.

When the event data is collated, the event data collection algorithm may summarize it to generate the one or more image generation instructions. These instructions may define, for example, the user's location when the emotion event occurred. Alternatively, or in addition, these instructions may define the user's emotional state during the emotion event. Alternatively, or in addition, these instructions may define what the user was doing when the emotion event occurred. Alternatively, or in addition, these instructions may define who the user was interacting with when the emotion event occurred. Alternatively, or in addition, these instructions may define specific details of the emotion event such as speech snippets and/or images.

Aspects of the one or more image generation instructions may be prioritized based on an algorithmic assessment by the event data collection algorithm such that key emotional and/or thematic features may be highlighted in a later coloring template 10. Summarization and/or prioritization of the event data to produce the one or more image generation instructions may be enabled using algorithmic text and/or other data summarization techniques that are known in the art.

As an example, the image generation algorithm may receive the emotion event and/or the event data and may be configured to generate an initial image based on the emotion event and/or the event data. Alternatively, or in addition, the image generation algorithm may receive the one or more image generation instructions and may be configured to generate the initial image based on their contents.

The properties of the (initial) image may vary. For example, the image may show real people, places and/or objects where the information is available. Alternatively, or in addition, the image may show stylized and/or generic representations of people, places and/or objects. Alternatively, or in addition, the image may show a blend of real and stylized images such as where the user has uploaded specific imagery to further personalize her/his experience of the system when real-time imagery is not available. For example, the user may upload images of themselves, her/his family members, locations she/he visits frequently and/or other features via the user interface. Alternatively, or in addition, the image may be generated in a particular artistic style as preferred by the user and/or selected by the computer system 200.

The image may be generated using algorithmic techniques known in the art such as e.g. using a generative adversarial network (GAN). For example, details from the one or more image generation instructions may be used by a generative algorithm configured to create an original image representing the emotion event. The keywords and/or other metadata from the one or more instructions may indicate to the algorithm which features to include and how to represent them. Alternatively, or in addition, details from the one or more image generation instructions may be used by a deepfake-style algorithm configured to find an existing image from online sources or a (proprietary) database matching the details of the emotion event and transfer personalized details from the user's experience into the image. As an example, the algorithm may transplant the user's face onto the image, as well as specific details about other people and objects as described by the one or more image generation instructions.

The size, orientation, file type and/or other standard settings of the image may be arbitrarily predefined e.g. based on the user's device type and/or interface requirements. As the image will form the basis of the coloring template 10, it may be generated in black and white or outline form. On the other hand, the coloring template 10 may comprise colored lines.

Generating 140 the coloring template 10 may comprise augmenting 141 at least one feature of the coloring template 10 relating to the excited level of the emotion of the user. As an example, and as in **Fig. 3-4****,** augmenting 141 at least one feature of the coloring template 10 relating to the excited level of the emotion of the user may be carried out by an image augmentation algorithm.

As an example, the (initial) image may be further adapted by the image augmentation algorithm, wherein the existing lines and boundaries of the image may be further augmented to produce an appropriate number and size of segments for the user to color in the final coloring template 10. The image augmentation algorithm may use the image generation instructions to determine the location and density of segments and coloring features such that the user may be induced to spend more time coloring specific sections. In so doing, the user's achieving mindfulness on particular features of the scene may be influenced and/or controlled. Additional segmentation and/or decorative highlighting may be applied to key features of the image as e.g. indicated by the event data collection algorithm. Further, a list of features that are associated with emotions and/or mindfulness may be predefined for additional segmentation. For example, segmentation may always be applied to faces, people, and/or activity indicating objects. Arbitrary constraints may be applied to ensure that coloring segments are designed at an appropriate scale for coloring (i.e. minimum and maximum segment area, length or width boundaries).

The method 100 may comprise providing 150 the coloring template 10 to the user. The coloring template 10 may be provided to the user via a user-interface of the computer system 200. As an example, and as in **Fig. 3****,** the user-interface may be of the coloring image generation system forming part of the computer system 200.

As an example, the coloring template 10 may be shared with the user via the user interface which may be used to view the one or more coloring templates. The user interface may also be used to print the coloring template 10 on a sheet of paper for manual (i.e. non-digital) coloring. Alternatively, or in addition, the user interface may be used to color the coloring template 10 using a digital interface such as a smart device (e.g. a tablet or smartphone).

The user interface may also be used for adjusting data collection settings of the event monitoring system. In case of digitally coloring in the coloring template 10, the user interface may also be used for accessing functions of the smart device on which the user is coloring the (digital) coloring template 10 to monitor the user during coloring. In so doing, coloring choices, such as colors, stroke speed, and/or pressure may be displayed and/or adjusted. Furthermore, the user interface may be used to display user responses to coloring, that is data inferred from (further) user data.

The method 100 may comprise obtaining 161 further user data generated by monitoring 160, via the user monitoring device, a further emotional state of the user while the user is coloring in the coloring template 10. The method 100 may further comprise obtaining 163 user action data generated by monitoring 162 the user's coloring in the coloring template 10.

The method 100 may or may not comprise monitoring 160, via the user monitoring device, the further emotional state of the user while the user is coloring in the coloring template 10. If the method 100 comprises monitoring 160, via the user monitoring device, the further emotional state of the user while the user is coloring in the coloring template 10, steps 160, 161 may be combined into a single step 160 (unlike in **Fig. 1b**): monitoring 160, via the user monitoring device, a further emotional state of the user while the user is coloring in the coloring template 10, thereby generating further user data.

The method 100 may or may not comprise monitoring 162 the user's coloring in the coloring template 10. If the method 100 comprises the user's coloring in the coloring template 10, steps 162, 163 may be combined into a single step (unlike in **Fig. 1b**): monitoring 162 the user's coloring in the coloring template 10, thereby generating user action data.

As an example, the user may color in the coloring template 10. The properties of the template may cause her/him to mindfully reflect on the event it depicts, with the segmentation of the coloring template 10 helping her/him to focus on specific emotional aspects of the event. During coloring, the user may be monitored to determine the effect of the coloring experience on their emotional state. User monitoring device(s) may be used to record (further) user data and/or user action data while the user colors the coloring template 10 to indicate the overall effect of the template. This may, in particular, apply to the case where the coloring template 10 is printed out on a sheet of paper. If the user monitoring device is a wearable, motion sensors in the device may be capable of monitoring information about the user's coloring technique by tracking the user's hand movements. For a digital coloring template 10, pressure and touch sensors in the tablet or other device being used to color the template may be used to track how the user colors and relate this to specific features of the coloring template 10.

The effect of the coloring template 10 on the user may be recorded and analyzed by the computer system 200 to select optimum features for subsequent and future coloring templates. Such recorded and analyzed data may function as training data with corresponding labels that may be beneficial in supervised learning. Hence, they may e.g. be used in adjusting 190 the algorithm configured to generate 140 the coloring template 10.

The method 100 may comprise guiding 170 the user through coloring in the coloring template 10, thereby generating at least one instruction to the user based on the further user data and/or the user action data. The at least one instruction to the user may be provided to the user via a user interface of, for example, the coloring image generation system. As an example, and as in **Fig. 3-4****,** guiding 170 the user through coloring in the coloring template 10 may be carried out by a guidance generation algorithm.

As an example, while the user interacts with the coloring template 10, her/his actions and (further) user data may be monitored to provide the user with guidance that may help her/him to color mindfully. The guidance generation algorithm may monitor the (further) user data as the user colors in the coloring template 10 e.g. on a smart tablet or similar device to assess the impact of the coloring template 10 on the user's mindfulness and/or stress. Changes in mindfulness or stress (e.g. indicated by changes in heart rate and/or attention) may be associated with regions and/or features of the coloring template 10. The user's coloring technique may also be monitored by the smart tablet upon which the user is completing the coloring template 10 to infer information about the user's state. For example, heavier pressure and/or more erratic strokes in a particular region may indicate that the user has a negative and/or other association with the feature being colored. Existing sensors in the device or stylus may be used to collect pressure and/or stroke information. Based on the user's current (further) user data (and/or user action data) and coloring technique, the guidance generation algorithm may generate feedback for the user (e.g. presented via the user interface) that is designed to improve the user's stress level and/or increase her/his mindfulness. Feedback may, for example, encourage the user to choose certain colors that may affect her/his stress level. Alternatively, or in addition, feedback may encourage the user to mindfully adjust her/his coloring technique (pressure, stroke speed, stroke length etc.) as they color in a certain area.

The method 100 may comprise adjusting 180 the coloring template 10 based on the further user data and/or on the user action data. In other words, the method 100 may comprise adjusting 180 the coloring template 10 while the user is coloring in the coloring template 10.

As an example, in case of a digital color template 10, the coloring template 10 may be adjusted dynamically as the user colors it to switch or maintain the user's focus on one or more certain features. As the user colors in the coloring template 10 on a tablet or similar device, the system may monitor her/his coloring technique and (further) user data to assess how the user interacts with the various segments and/or features of the coloring template 10. Based on how the segments and/or features affect the (further) user data, the image augmentation algorithm or another algorithm may dynamically adapt the coloring template 10. For example, the number of segments in an image may be altered to increase or reduce the use of a certain color associated with those regions that may be effective at reducing stress. Alternatively, or in addition, the number of segments in an image may be altered to increase or reduce the use of a certain stroke length and/or style that is impacting the user's mindfulness state positively or negatively. Alternatively, or in addition, the number of segments around a specific feature may be altered to maintain the user's focus on that feature for a longer time. The adapted coloring template 10 may be seamlessly incorporated into the user's coloring experience so that the user can continue coloring while the changes are being made.

The method 100 may comprise adjusting 190 an algorithm configured to generate 140, based on the emotion event, the coloring template 10 based on the further user data and/or on the user action data. As an example, the algorithm to be adjusted 190 may be the image generation algorithm. Alternatively, or in addition, the algorithm to be adjusted 190 may be the image augmentation algorithm. Alternatively, or in addition, the algorithm to be adjusted 190 may be the guidance generation algorithm. Alternatively, or in addition, the algorithm to be adjusted 190 may be the event data collection algorithm.

There is disclosed a computer system 200 configured to execute the computer-implemented method 100 for generating a personalized coloring template 10 for a user. The computer system 200 may comprise at least one processor such as e.g. a (C)PU and a memory such as e.g. RAM The computer system 200 may further comprise a storage such as e.g. HDD or SDD. The computer system 200 may be configured for data exchange with a (cloud) server. The computer system 200 may comprise (or be) the coloring image generation system, as e.g. in **Fig. 3****.** The computer system 200 may or may not comprise the event monitoring system. In particular, the computer system 200 may or may not comprise the user monitoring device. In case the event monitoring system and/or the user monitoring device are not comprised by the computer system 200, the computer system 200 may be configured to couple to these systems. Unlike displayed in **Fig. 3****,** the emotion analysis algorithm and/or the event data collection algorithm may be implemented in the coloring image generation system.

As an example, the computer system 200 may comprise a system (e.g. referred to as the event monitoring system) configured to monitor a user's daily experiences and identify key events that may positively or negatively affect a user's mental health. The event monitoring system may comprise one or more devices (e.g. referred to as user monitoring device(s)) capable of and configured for monitoring and recording physiological data associated with a user's emotional responses (e.g. referred to as the user data). The event monitoring system may be configured to implement an algorithm (e.g. referred to as the emotion analysis algorithm) configured to identify when a notable emotional event has occurred (e.g. referred to as emotion event) and extract the user's associated emotional state from the collected user data. The event monitoring system may be configured to implement an algorithm (e.g. referred to as the event data collection algorithm) configured to collect relevant data (e.g. referred to as the event data) relating to a user's daily life that is associated with a specific emotion event.

Event data may comprise, for example, location data (e.g. GPS) indicating where the user was when the emotion event took place. Alternatively, or in addition, event data may comprise Bluetooth-enabled nearby user data. Alternatively, or in addition, event data may comprise activity data detected from user movements. Alternatively, or in addition, event data may comprise schedule data. Alternatively, or in addition, event data may comprise real-time recordings of images. Alternatively, or in addition, event data may comprise sound data. Alternatively, or in addition, event data may comprise other data.

For example, event data may be collected from various sources (e.g. referred to as the data sources in **Fig. 4**). Event data may be collected from one or more sensors of the computer system 200. In particular, event data may be collected from the user monitoring device(s). The one or more sensors may or may not be part of the coloring image generation system. Alternatively, or in addition, event data may be collected from digital journaling and/or reflection services. Alternatively, or in addition, event data may be collected from one or more "always on" smart home devices. Alternatively, or in addition, event data may be collected from digital calendars and/or planning services. Alternatively, or in addition, event data may be collected from an online source, that is from a server. Alternatively, or in addition, event data may be collected from manual user entry of events and images.

Furthermore, event data may be added manually by the user and/or a medical professional (such as an art therapist or psychiatrist). The event data collection algorithm may be configured to summarize the event data into a set of instructions (e.g. referred to as the image generation instructions) which may be used to define the required features of a coloring template 10 to be generated.

As an example, the computer system 200 may comprise a system (e.g. referred to as the coloring image generation system) configured to generate a personalized coloring template (that is, the coloring template 10) for a user based on her/his daily experiences and associated emotional state.

The coloring image generation system may implement an algorithm (e.g. referred to as the image generation algorithm) configured to generate an initial image (e.g. referred to as the image) based on a set of image generation instructions. The image may be generated using algorithmic techniques such as e.g. machine learning algorithms pre-trained on such a task. Such a machine learning algorithm may, for example, be a generative adversarial network (GAN). As examples, the image may show people, locations, objects and/or other features relevant to the image generation instructions. Representations of the user and/or others in the image may be created such that the facial expressions and body posture of the characters reflect the emotions described by the emotion event (and included in the image generation instructions). Where appropriate, the image generation algorithm may draw on online sources to populate the image. For example, where a specific location is identified in the image generation instructions, the image generation algorithm my access a photograph and/or other details of that location to inform how the image may be generated.

Furthermore, the coloring image generation system may implement an algorithm (e.g. referred to as the image augmentation algorithm) to augment the appearance of the image to produce the coloring template appropriate for mindful coloring. For example, the image augmentation algorithm may adjust the image by segmenting sections of the image into smaller bounded regions for coloring. Alternatively, or in addition, the image augmentation algorithm may adjust the image by adding decorative, stylized and/or other aesthetically relevant features to the image. As examples, the image augmentation algorithm may adjust the image by e.g. floral patterns, fractals, stars, shapes, scenery, symbols and/or etc. An example floral pattern is displayed at the woman's head in the coloring template 10 displayed in **Fig. 5****.**

The image augmentation algorithm may be configured to add one or more features to the image in a targeted manner, such that the location of the features causes the user to spend more time focusing on a particular region of the coloring template 10. This may be used to induce the user to spend more time focusing on certain regions that may benefit from mindful reflection. Such a region may, for example, be a particular feature, such as a face and/or object key to the emotion event. Alternatively, or in addition, such a region may display a particular emotion associated with a feature or person.

The computer system 200 may be configured to allow the user to color in the coloring template 10 digitally. In this case, the image augmentation algorithm (or another such algorithm) may dynamically adjust the properties of the coloring template as the user colors it in, in response to real-time user data.

Furthermore, the coloring image generation system may comprise a user interface through which the user may, for example, interact with the coloring template(s) 10. In general, the user interface may be configured to allow the user to interact with the computer system 200 and/or with the computer-implemented method 100. As an example, preferences for image augmentation via the image augmentation algorithm may be set via the user interface. Likewise preferences for any algorithm of method 100 may be set.

Furthermore, the coloring image generation system may implement an algorithm (e.g. referred to as the guidance generation algorithm) to generate mindful coloring guidance for the user based on her/his user data response to the activity. The guidance generation algorithm may be used in the case where the coloring template is digitally colored in by the user. For example, the smart device on which the user is completing her/his digital coloring experience may be configured to monitor and provide feedback on the experience. On the other hand, guidance may also be provided in a manual coloring scenario (that is on a sheet of paper) from a smart assistant or similar, where simple instructions based on the user's physiological response to the coloring may be provided.

The guidance generation algorithm may be configured to record the effect of specific guidance on the user data in a database (e.g. referred to as the user effect database) for future sessions, such that the computer system 200 may learn the optimum colors, stroke technique and/or feature segmentation to improve the user's stress or mindfulness state.

There is disclosed a computer program configured to execute the computer-implemented method 100 for generating a personalized coloring template 10 for a user. The computer program may be in interpretable or compiled form. The computer program or portions thereof may be loaded as a bit or byte sequence into the RAM of the computer system 200.

There may be disclosed a computer-readable medium or signal storing the computer program. The medium may be e.g. one of RAM, ROM, EPROM, HDD, SDD etc. storing the computer program.

Alternatively to method 100 or in addition, it may be beneficial for event data to be added manually by the user and/or a medical professional. The user may experience an event that they specifically want to mindfully reflect on and may share information about the event with the event data collection algorithm. Alternatively, or in addition, a medical professional may be aware of specific themes or events that the user would benefit from reflecting on as part of a therapy treatment or similar. The medical professional may upload suggestions to e.g. the event data collection algorithm that define specific themes, people, places and emotions. In this scenario, the user's interaction with the therapist-defined coloring template may be monitored via one or more user monitoring devices as described for method 100. The user's stress response/mindfulness data may then be shared with the therapist with the user's permission, to better inform future therapy sessions and/or therapist coloring template suggestions. In both cases, the user interface may be used to facilitate manual upload of event data. Event data may be shared in various forms as described in the method 100. Alternatively, or in addition, event data may be manually added by, for example, the user providing a brief textual description to the system which may be interpreted using natural language processing technologies that are known in the art. Alternatively, or in addition, event data may be manually added by, for example, the user selecting scenarios or features of a scenario from a pre-set list of locations, objects, people and/or other aspects.

Although the present invention has been described above and is defined in the attached claims, it should be understood that the invention may alternatively be defined in accordance with the following embodiments:
1. A computer-implemented method (100) for generating a personalized coloring template for a user of a user monitoring device, comprising:
   - obtaining (111) user data generated by monitoring (110) the user's emotional state via the user monitoring device;
   - obtaining (121) an emotion event generated by detecting (120) an excited level of an emotion of the user based on the user data;
   - generating (140) a coloring template (10) based on the emotion event, thereby generating the personalized coloring template for the user.
2. The method (100) of embodiment 1, wherein obtaining (111) the user data generated by monitoring (110) the user's emotional state via the user monitoring device comprises monitoring (110) the user's emotional state via the user monitoring device, thereby generating the user data.
3. The method (100) of embodiment 1 or 2, wherein obtaining (121) the emotion event generated by detecting (120) the excited level of the emotion of the user based on the user data comprises detecting (120) the excited level of the emotion of the user based on the user data, thereby generating the emotion event.
4. The method (100) of one of the preceding embodiments, comprising:
   - obtaining (131) event data generated from gathering (130) context information relating to the emotion event;
   wherein generating (140) the coloring template (10) is further based on the event data.
5. The method (100) of embodiment 4, wherein gathering (130) the context information relating to the emotion event comprises collecting context information relating to the emotion event via the user monitoring device.
6. The method (100) of embodiment 4 or 5, wherein gathering (130) the context information relating to the emotion event comprises recording context information relating to the emotion event by at least one sensor system.
7. The method (100) of one of the preceding embodiments, comprising:
   - generating (139) at least one image generation instruction based on the emotion event and/or the event data;
   wherein generating (140) the coloring template (10) is further based on the at least one image generation instruction.
8. The method (100) of one of the preceding embodiments, wherein generating (140) the coloring template (10) comprises augmenting (141) at least one feature of the coloring template (10) relating to the excited level of the emotion of the user.
9. The method (100) of one of the preceding embodiments, comprising:
   - providing (150) the coloring template (10) to the user.
10. The method (100) of one of the preceding embodiments, wherein detecting (120) the excited level of the emotion of the user comprises:
   - applying the user data or a portion thereof relating to a current point in time to a predetermined criterion;
   - detecting the excited level of the emotion of the user relating to the current point in time, if the predetermined criterion is satisfied.
11. The method (100) of embodiment 10, wherein the user data comprises one or more of a heart rate of the user, a galvanic skin response of the user, an electroencephalography of the user, and/or a facial expression of the user, and wherein the excited level of the emotion of the user is detected (120), if one or more of a heart rate of the user, a galvanic skin response of the user, an electroencephalography of the user, and/or a facial expression of the user satisfy the predetermined criterion.
12. The method (100) of one of the preceding embodiments, comprising:
   - obtaining (161) further user data generated by monitoring (160), via the user monitoring device, a further emotional state of the user while the user is coloring in the coloring template (10); and
   - obtaining (163) user action data generated by monitoring (162) the user's coloring in the coloring template (10).
13. The method (100) of embodiment 12, comprising:
   - guiding (170) the user through coloring in the coloring template (10), thereby generating at least one instruction to the user based on the further user data and/or the user action data.
14. The method (100) of embodiment 12 or 13, comprising:
   - adjusting (180) the coloring template (10) based on the further user data and/or on the user action data.
15. The method (100) of one of the embodiments 12 to 14, comprising:
   - adjusting (190) an algorithm configured to generate (140), based on the emotion event, the coloring template (10) based on the further user data and/or on the user action data.
16. A computer system (200) configured to execute the computer-implemented method (100) for generating a personalized coloring template (10) for a user according to one of the preceding embodiments.
17. A computer program configured to execute the computer-implemented method (100) for generating a personalized coloring template (10) for a user according to one of the embodiments 1 to 15.
18. A computer-readable medium or signal storing the computer program of embodiment 17.

## Claims

1. A computer-implemented method (100) for generating a personalized coloring template for a user of a user monitoring device, comprising:
- obtaining (111) user data generated by monitoring (110) the user's emotional state via the user monitoring device;
- obtaining (121) an emotion event generated by detecting (120) an excited level of an emotion of the user based on the user data;
- generating (140) a coloring template (10) based on the emotion event, thereby generating the personalized coloring template for the user.

2. The method (100) of claim 1, wherein obtaining (111) the user data generated by monitoring (110) the user's emotional state via the user monitoring device comprises monitoring (110) the user's emotional state via the user monitoring device, thereby generating the user data.

3. The method (100) of claim 1 or 2, wherein obtaining (121) the emotion event generated by detecting (120) the excited level of the emotion of the user based on the user data comprises detecting (120) the excited level of the emotion of the user based on the user data, thereby generating the emotion event.

4. The method (100) of one of the preceding claims, comprising:
- obtaining (131) event data generated from gathering (130) context information relating to the emotion event;
wherein generating (140) the coloring template (10) is further based on the event data.

5. The method (100) of claim 4, wherein gathering (130) the context information relating to the emotion event comprises collecting context information relating to the emotion event via the user monitoring device.

6. The method (100) of claim 4 or 5, wherein gathering (130) the context information relating to the emotion event comprises recording context information relating to the emotion event by at least one sensor system.

7. The method (100) of one of the preceding claims, wherein generating (140) the coloring template (10) comprises augmenting (141) at least one feature of the coloring template (10) relating to the excited level of the emotion of the user.

8. The method (100) of one of the preceding claims, comprising:
- providing (150) the coloring template (10) to the user.

9. The method (100) of one of the preceding claims, wherein detecting (120) the excited level of the emotion of the user comprises:
- applying the user data or a portion thereof relating to a current point in time to a predetermined criterion;
- detecting the excited level of the emotion of the user relating to the current point in time, if the predetermined criterion is satisfied.

10. The method (100) of claim 9, wherein the user data comprises one or more of a heart rate of the user, a galvanic skin response of the user, an electroencephalography of the user, and/or a facial expression of the user, and wherein the excited level of the emotion of the user is detected (120), if one or more of a heart rate of the user, a galvanic skin response of the user, an electroencephalography of the user, and/or a facial expression of the user satisfy the predetermined criterion.

11. The method (100) of one of the preceding claims, comprising:
- obtaining (161) further user data generated by monitoring (160), via the user monitoring device, a further emotional state of the user while the user is coloring in the coloring template (10); and
- obtaining (163) user action data generated by monitoring (162) the user's coloring in the coloring template (10).

12. The method (100) of claim 11, comprising:
- guiding (170) the user through coloring in the coloring template (10), thereby generating at least one instruction to the user based on the further user data and/or the user action data.

13. The method (100) of claim 11 or 12, comprising:
- adjusting (180) the coloring template (10) based on the further user data and/or on the user action data.

14. The method (100) of one of the claims 11 to 13, comprising:
- adjusting (190) an algorithm configured to generate (140), based on the emotion event, the coloring template (10) based on the further user data and/or on the user action data.

15. A computer system (200) configured to execute the computer-implemented method (100) for generating a personalized coloring template (10) for a user according to one of the preceding claims.
